(19) Europäisches Patentamt
European Patent Office
Office européen des brevets

(11) Numéro de publication : **0 387 322 B1**

(12) **FASCICULE DE BREVET EUROPEEN**

(45) Date de publication du fascicule du brevet : **31.08.94 Bulletin 94/35**

(51) Int. Cl.$^5$ : **G02F 1/35,** C07D 333/24, C07D 307/54

(21) Numéro de dépôt : **89909205.0**

(22) Date de dépôt : **20.07.89**

(86) Numéro de dépôt international : **PCT/FR89/00382**

(87) Numéro de publication internationale : **WO 90/01724 22.02.90 Gazette 90/05**

# (54) MATERIAU ORGANIQUE POUR L'OPTIQUE NON LINEAIRE.

(30) Priorité : **01.08.88 FR 8810373**

(43) Date de publication de la demande : **19.09.90 Bulletin 90/38**

(45) Mention de la délivrance du brevet : **31.08.94 Bulletin 94/35**

(84) Etats contractants désignés : **DE FR GB IT NL**

(56) Documents cités :
**EP-A- 0 220 042**
**D.J. Williams, Angew. Chem. Int. Ed. Engl. Vol. 23 (1984), pages 690-703**
**Patent Abstracts of Japan, volume 12, Nr. 334 (P-756) (3181), 8 September 1988; & JP A 6396639**
**J. OPT. SOC. Am. B, vol. 4, Nr. 6, June 1987 Optical Soc. of America (US) G. Marowsky et al.: "Efficiency studies of second-harmonic-active organic dye coverages", pp 956-961.**

(73) Titulaire : **ALCATEL N.V.**
**Strawinskylaan 341,**
**(World Trade Center)**
**NL-1077 XX Amsterdam (NL)**

(72) Inventeur : **FAUVARQUE, Jean-François**
**6, rue Joseph-Bara**
**F-75006 Paris (FR)**
Inventeur : **RATOVELOMANANA, Victorien**
**Laboratoire Lecso**
**C.N.R.S.**
**2, rue Henry-Dunant F-94320 Thiais (FR)**
Inventeur : **JUTAND, Anny**
**25, rue Mouton-Duvernet**
**F-75014 Paris (FR)**
Inventeur : **AMATORE, Christian**
**39, rue Dunois**
**F-75013 Paris (FR)**

(74) Mandataire : **Weinmiller, Jürgen**
**Lennéstrasse 9**
**Postfach 24**
**D-82336 Feldafing (DE)**

**EP 0 387 322 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un matériau organique pour l'optique non linéaire.

Les matériaux présentant des propriétés non linéaires en optique peuvent être utilisés pour de nombreuses applications : doubleurs de fréquence, bistables optiques, commutateurs, modulateurs, coupleurs directifs, amplificateurs paramétriques etc...

On connaît un petit nombre de matériaux inorganiques non isotropes, et en particulier non centrosymétriques, qui possèdent un coefficient quadratique non nul et des propriétés non linéaires en optique ; il s'agit notamment du potassium-dihydrogène-phosphate (KDP), du niobate et du tantalate de lithium ($LiNbO_3$, $LiTaO_3$)...

La mise en oeuvre de ces matériaux inorganiques est souvent difficile ; c'est pourquoi on s'est intéressé à des matériaux organiques à propriétés non linéaires, en particulier quand il s'agit de matériaux polymériques, thermoplastiques ou filmogènes.

La non linéarité optique est généralement obtenue en incorporant, par mélange ou greffage chimique dans le matériau organique des molécules ou groupements moléculaires polaires fortement hyperpolarisables.

De telles molécules sont synthétisées en associant un groupe attracteur d'électrons à un groupe donneur d'électrons par l'intermédiaire d'un système transmetteur d'effets électroniques.

On connaît par exemple les matériaux suivants :

- La paranitroaniline (PAN)

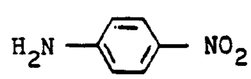

- Le N (nitro 4 phényl) N méthyl amino 2 acétonitrile (NPAN)

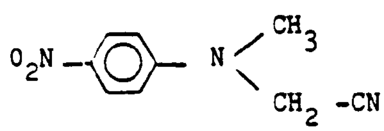

- La dinitro 2-4 phényl L alanine (MAP)

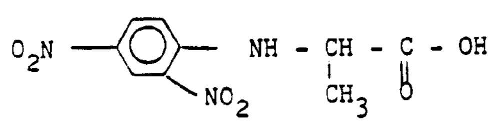

- Le méthyl 3 nitro 4 pyridine N-oxyde (POM)

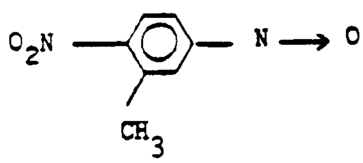

L'article de D.J. Williams "Angew. Chem. Int. Ed. Engl." 23 pp 690-703 divulgue un matériau organique comprenant un groupe attracteur d'électrons et un groupe donneur d'électrons reliés par un transmetteur d'électrons, et en particulier les combinaisons d'un phényle comme transmetteur d'électrons avec un nitro ou un cyano comme groupe attracteur ainsi qu'une chaîne de deux atomes de carbone présentant une double liaison comme transmetteur d'électrons. Comme groupe attracteur, on utilise un nitro et le groupe donneur est un dérivé du méthyle ou un amine.

Ces molécules sont caractérisées par leur coefficient d'hyperpolarisabilité β défini par le développement

en série du moment dipolaire $\vec{\mu}$ en fonction du champ électrique E.

$$\vec{\mu} = \vec{\mu}_0 + \alpha.\vec{E} + \beta|\vec{E}|^2 + \ldots$$

Le coefficient β peut être mesuré par la méthode EFISH (Electric Field Induced Second Harmonic generation).

Beaucoup d'auteurs se basent sur la valeur de l'hyperpolarisabilité moléculaire associée au doublement de fréquence infrarouge pour juger de la valeur d'une molécule synthétisée.

Pour ces applications, des molécules à grand nombre d'atomes peuvent convenir, mais l'expérience montre que de telles molécules absorbent fortement la lumière dans le spectre visible, ce qui peut être gênant ; pour les applications électro-optiques, la grandeur utile est plutôt l'hyperpolarisabilité par unité de masse ou par unité de volume.

La présente invention a pour but de rechercher des molécules comportant tous les éléments essentiels (groupe attracteur, groupe donneur et groupe transmetteur) avec le plus petit nombre possible d'atomes, tout on conservant une bonne valeur d'hyperpolarisabilité moléculaire à champ constant.

Ce but est atteint selon l'invention par le matériau tel que défini dans la revendication 1.

L'atome d'azote peut être lié à un radical R choisi parmi H, $CH_3$, $C_2H_5$.

Selon une première variante, ledit groupe transmetteur est formé par une liaison éthényle.

A titre d'exemple le matériau selon l'invention peut répondre à l'une des formules suivantes :

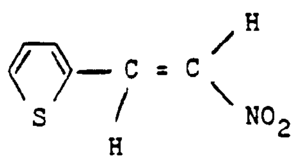

(transnitrovinylidène) - 2 thiophène

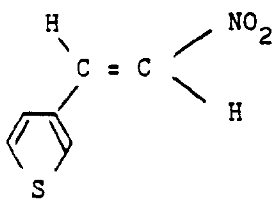

(transnitrovinylidène) - 3 thiophène

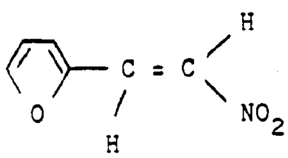

(transnitrovinylidène) - 2 furanne

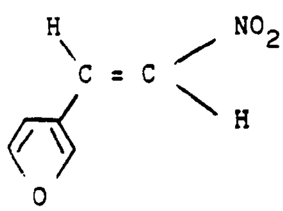

(transnitrovinylidène) - 3 furanne.

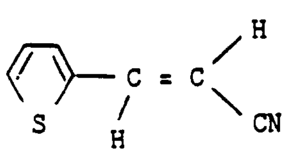

(transcyanovinylidène) - 2 thiophène

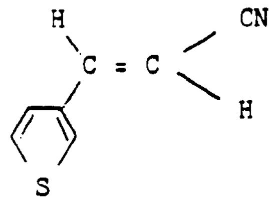

(transcyanovinylidène) - 3 thiophène

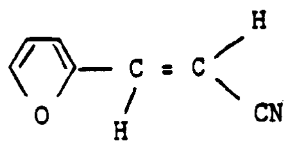

(transcyanovinylidène) - 2 furanne

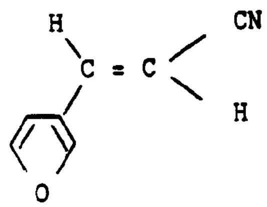

(transcyanovinylidène) - 3 furanne.

Selon une seconde variante, ledit groupe transmetteur est formé par un noyau phényle. Le matériau selon l'invention peut répondre alors à l'une des formules suivantes :

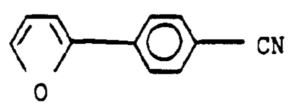

(cyano 4 phényl) - 2 furanne

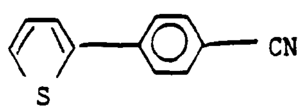

(cyano 4 phényl) - 2 thiophène

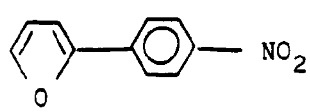

(nitro 4 phényl) - 2 furanne

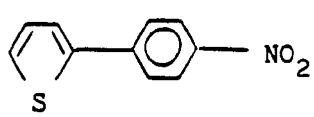

(nitro 4 phényl) - 2 thiophène

Selon une autre variante ledit cycle hétéroaromatique est substitué en 5 par un groupement choisi parmi $CH_3O$, $CH_3S$, $NH_2$, $CH_3NH$, $(CH_3)_2N$.

Dans toutes les molécules selon l'invention le caractère donneur d'électrons et le caractère aromatique sont concentrés en un plus petit nombre d'atomes que dans les molécules antérieures car elles contiennent des cycles aromatiques riches en électrons (furanne, thiophène, pyrrole substitués ou non).

Avec ces molécules possédant un petit nombre d'atomes, des hyperpolarisabilités moléculaires élevées à fréquence nulle peuvent être atteintes.

D'excellents résultats peuvent être obtenus en utilisant le groupe "cyano" (-CN) plus petit que le groupe

"nitro ($-NO_2$).

La présente invention a également pour objet des procédés de synthèse des matériaux précédents.

D'autres caractéristiques et avantages de la présente invention apparaîtront au cours de la description suivante de modes de réalisation donnés à titre illustratif mais nullement limitatif.

EXEMPLE 1

On prépare du (transnitrovinylidène) - 2 thiophène

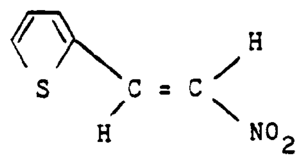

Dans un tricol de 250 cm³ équipé d'un thermomètre, d'une ampoule à brome, et d'un septum, on place 5,60 g de thiophène - 2 carbaldéhyde, 3,55 g de nitrométhane, 1,01 g d'aliquat 336 (Aldrich) et 100 ml de méthanol. On refroidit vers 0-5°C.

Sous agitation magnétique, on verse goutte à goutte a l'aide de l'ampoule à brome 10 ml de soude 10N en veillant à ce que la température ne monte pas au dessus de 5°C.

On agite encore 10 minutes après la fin de l'addition de la soude. Puis on transvase le tout goutte à goutte dans 50 ml d'une solution d'acide chlorhydrique 4N.

Le tout est ensuite conservé à 4°C pendant 24 heures. Il se forme des cristaux qui sont filtrés et lavés à l'eau distillée et séchés pendant 36 heures.

On obtient ainsi 6,9 g de produit, soit un rendement de 89%. Le produit est ensuite purifié par filtration sur silice.

EXEMPLE 2

On prépare du (transnitrovinylidène) - 3 thiophène

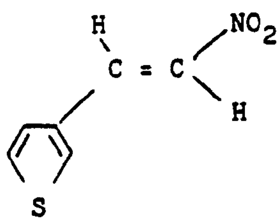

La procédure précédente est reproduite à partir de 5,4 g de thiophène - 3 carbaldéhyde. On obtient 6,6 g de produit soit 85% de rendement.

EXEMPLE 3

On prépare du (transnitrovinylidène) - 2 furanne

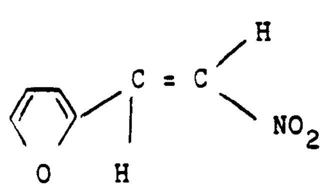

La procédure précédente est appliquée à 4, 8 g de furfuraldéhyde. On obtient 5,02 g de produit, c'est-à-dire 72% de rendement.

EXEMPLE 4

On prépare du (transnitrovinylidène) - 3 furanne

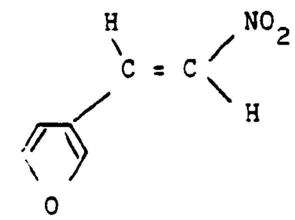

La procédure précédente est appliquée à 4,8 g du furanne -3 carbaldéhyde. On obtient 5,7 g de produit, c'est-à-dire 82% de rendement.

Les quatre matériaux analogues aux quatre précédents, mais où le groupe nitro a été remplacé par un groupe cyano, peuvent être préparés de manière analogue.

EXEMPLE 5

On prépare du (cyano 4 phényl) - 2 furanne

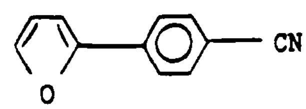

Le bromo -2- furanne n'étant pas commercial, le dérivé zincique correspondant est préparé en traitant dans 50 ml de tétrahydrofuranne (THF) 5 m. moles de furanne par 6 m. moles de butyllithium.
La solution obtenue est ensuite ajoutée à une solution de 6 m. moles de chlorure de zinc $ZnCl_2$ dans 60 ml de THF. On fait ensuite réagir le zincique obtenu sur 5 m. moles de parabromobenzonitrile en présence du complexe de palladium Pd $[P(C_6H_5)_3]_4$. On obtient le (cyano 4 phényl) - 2 furanne avec 74% de rendement de produit isolé.

L'absorption de ce matériau est maximale à 304 nanomètres, son coefficient d'absorption moléculaire ε étant alors de 48.740. Ce dernier est voisin de zéro à 400 nanomètres. Sa transparence optique est parfaite dans le domaine de fréquences du spectre visible et proche infrarouge. Il peut donc être utilisé pour générer un second harmonique dans la région bleue du spectre visible à partir d'un laser infrarouge ou proche infrarouge.

EXEMPLE 6

On prépare du (cyano 4 - phényle) - 2 thiophène

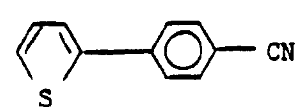

Pour cela on prépare le dérivé magnésien du bromo 2 thiophène (5 m. moles) dans 50 ml de THF; on fait réagir le dérivé magnésien avec le paraiodobenzonitrile et en présence du complexe de palladium Pd $[P(C_6H_5)_3]_4$. On obtient le (cyano 4 - phényle) - 2 thiophène avec 73% de rendement en produit isolé.

Ce matériau présente un maximum d'absorption à 302 nanomètres, son coefficient d'absorption moléculaire ε étant de 87.623. Ce dernier est voisin de 0 à 400 nanomètres. Il présente les mêmes avantages que le produit de l'exemple 5.

EXEMPLE 7

De manière analogue le (nitro 4 phényl) - 2 furanne peut être obtenu à partir de

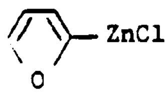 et de 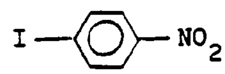

Son maximum d'absorption se situe à 342 nanomètres avec un coefficient d'absorption ε de 16.666 ; ce dernier passe à 1935 pour 400 nanomètres.

EXEMPLE 8

De même, le (nitro 4 phényl) - 2 thiophène peut être obtenu à partir de

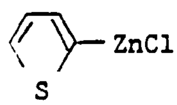 et de 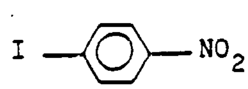

Son maximum d'absorption se situe à 344 nanomètres avec un coefficient d'absorption ε de 15.537 ; ce dernier passe à 1624 à 400 nanomètres.

Les exemples 5 à 8 montrent que d'excellents résultats peuvent être obtenus en utilisant un groupe attracteur "cyano" (-CN) plus petit qu'un groupe "nitro" (-$NO_2$).

L'utilisation des dérivés cyanés se révèle particulièrement avantageuse en lumière visible, car ces produits absorbent beaucoup moins cette lumière que les dérivés nitrés correspondants.

Bien entendu l'invention n'est pas limitée aux seuls exemples décrits, ni aux modes de synthèse mentionnés.

**Revendications**

**1.** Matériau organique possédant des propriétés optiques non linéaires, et dont la molécule est formée :
- d'un groupe donneur d'électrons comprenant un cycle hétéroaromatique comportant dans sa chaîne quatre atomes de carbone et un atome choisi parmi S, O et N
- d'un groupe attracteur d'électrons choisi parmi $NO_2$ et CN,
- d'un groupe transmetteur d'électrons choisi parmi une chaîne à n atomes de carbone (avec $n < 4$) et présentant une double ou une triple liaison, et un système aromatique tel qu'un noyau phényle, ledit groupe transmetteur d'électrons étant lié audit groupe donneur d'électrons par l'un desdits quatre atomes de carbone.

**2.** Matériau organique selon la revendication 1, caractérisé par le fait que l'atome d'azote est lié à un radical R choisi parmi H, $CH_3$, $C_2H_5$.

**3.** Matériau selon l'une des revendications 1 et 2, caractérisé par le fait que ledit groupe transmetteur est formé par une liaison éthényle.

**4.** Matériau selon la revendication 3, caractérisé par le fait qu'il répond à l'une des formules suivantes :

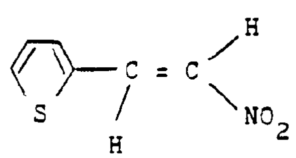 (transnitrovinylidène) - 2 thiophène

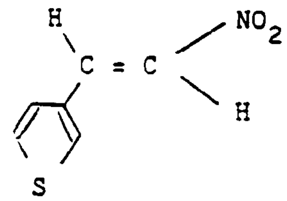

(transnitrovinylidène) - 3 thiophène

5. Matériau selon la revendication 3, caractérisé par le fait qu'il répond à l'une des formules suivantes :

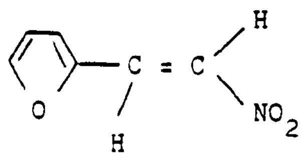

(transnitrovinylidène) - 2 furanne

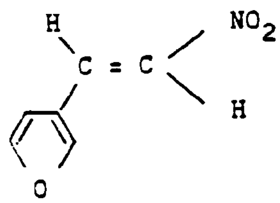

(transnitrovinylidène) - 3 furanne.

6. Matériau selon la revendication 3, caractérisé par le fait qu'il répond à l'une des formule suivantes :

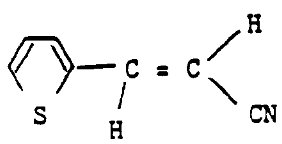

(transcyanovinylidène) - 2 thiophène

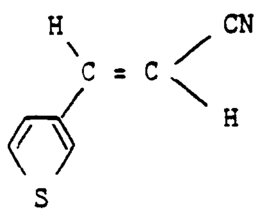

(transcyanovinylidène) - 3 thiophène

7. Matériau selon la revendication 3, caractérisé par le fait qu'il répond à l'une des formules suivantes :

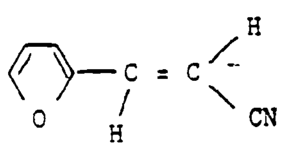

(transcyanovinylidène) - 2 furanne

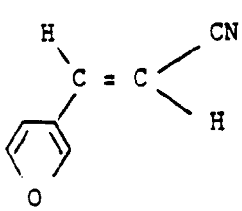

(transcyanovinylidène) - 3 furanne.

8. Matériau selon la revendication 1, caractérisé par le fait qu'il répond à la formule suivante :

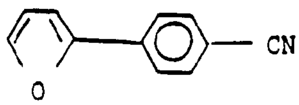 (cyano 4 phényl) - 2 furanne

**9.** Matériau selon la revendication 1, caractérisé par le fait qu'il répond à la formule suivante :

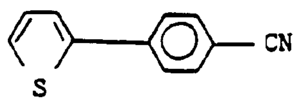 (cyano 4 phényl) - 2 thiophène

**10.** Matériau selon la revendication 1, caractérisé par le fait qu'il répond à la formule suivante :

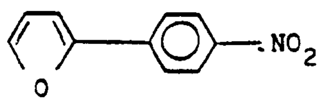 (nitro 4 phényl) - 2 furanne

**11.** Matériau selon la revendication 1, caractérisé par le fait qu'il répond à la formule suivante :

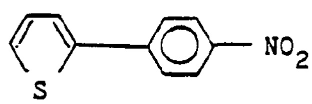 (nitro 4 phényl) - 2 thiophène

**12.** Matériau selon la revendication 1, caractérisé par le fait que ledit cycle hétéroaromatique est substitué en 5 par un groupement choisi parmi $CH_3O$, $CH_3S$, $NH_2$, $CH_3NH$, $(CH_3)_2N$.

**13.** Méthode de préparation du matériau selon la revendication 8, caractérisée par le fait que l'on couple le dérivé zincique

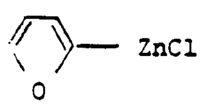

au parabromobenzonitrile

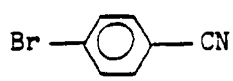

en présence de quantités catalytiques de complexes du palladium.

**14.** Méthode de préparation du matériau selon la revendication 9, caractérisée par le fait que l'on couple le dérivé magnésien

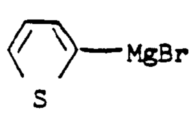

au paraiodobenzonitrile

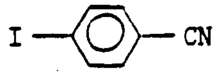

en présence de quantités catalytiques de complexes du palladium.

**15.** Méthode de préparation du matériau selon la revendication 10, caractérisé par le fait que l'on couple le dérivé zincique

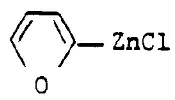

au paraiodonitrobenzène

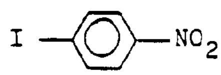

en présence de quantités catalytiques de complexe du palladium.

**16.** Méthode de préparation du matériau selon la revendication 11, caractérisé par le fait que l'on couple le dérivé zincique

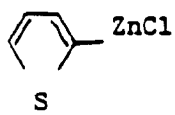

au paraiodonitrobenzène

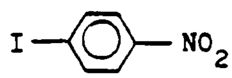

en présence de quantités catalytiques de complexes du palladium.

## Patentansprüche

**1.** Organisches Material mit nichtlinearen optischen Eigenschaften, dessen Molekül gebildet wird
- aus einer Elektronen abgebenden Gruppe, die einen heteroaromatischen Zyklus mit vier Kohlenstoffatomen in der Kette und einem unter S, O und N ausgewählten Atom enthält,
- einer Elektronen anziehenden Gruppe, die aus $NO_2$ und CN ausgewählt wird,
- einer Elektronen übertragenden Gruppe, die unter einer Kette von n Kohlenstoffatomen (mit $n < 4$) ausgewählt wird und eine doppelte oder dreifache Bindung sowie ein aromatisches System wie z.B. einen Phenylkern besitzt, wobei die Elektronen übertragende Gruppe mit der Elektronen abgebenden Gruppe über eines der vier Kohlenstoffatome gebunden ist.

**2.** Organisches Material nach Anspruch 1, dadurch gekennzeichnet, daß das Stickstoffatom an ein Radikal R gebunden ist, das unter H, $CH_3$, $C_2H_5$ ausgewählt wird.

**3.** Material nach einem der Ansprüche 1 und 2, dadurch gekennzeichnet, daß die Elektronen übertragende Gruppe von einer Ethenyl-Bindung gebildet wird.

**4.** Material nach Anspruch 3, dadurch gekennzeichnet, daß es einer der folgenden Formeln entspricht:

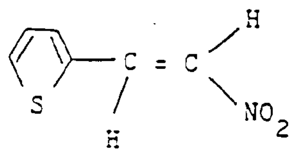

(Transnitrovinyliden)-2-thiophen

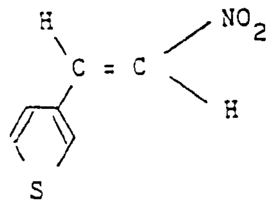

(Transnitrovinyliden)-3-thiophen

**5.** Material nach Anspruch 3, dadurch gekennzeichnet, daß es einer der folgenden Formeln entspricht:

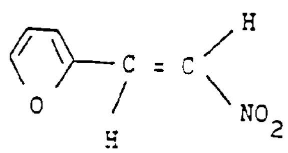

(Transnitrovinyliden)-2-furan

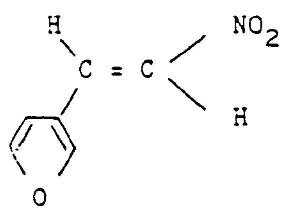

(Transnitrovinyliden)-3-furan

**6.** Material nach Anspruch 3, dadurch gekennzeichnet, daß es einer der folgenden Formeln entspricht:

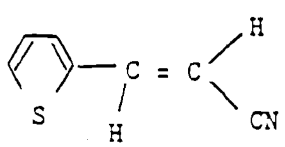

(Transcyanovinyliden)-2-thiophen

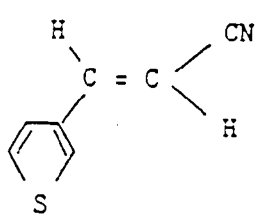

(Transcyanovinyliden)-3-thiophen

**7.** Material nach Anspruch 3, dadurch gekennzeichnet, daß es einer der folgenden Formeln entspricht:

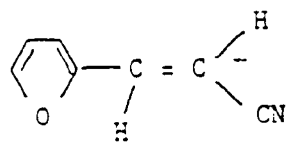

(Transcyanovinyliden)-2-furan

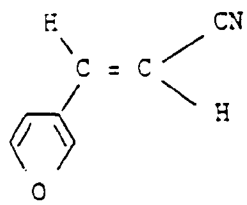

(Transcyanovinyliden)-3-furan

**8.** Material nach Anspruch 1, dadurch gekennzeichnet, daß es der folgenden Formel entspricht:

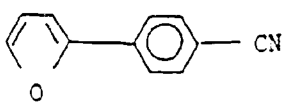

(Cyano-4-phenyl)-2-furan

**9.** Material nach Anspruch 1, dadurch gekennzeichnet, daß es der folgenden Formel entspricht:

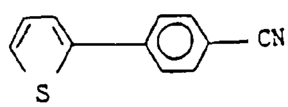

(Cyano-4-phenyl)-2-thiophen

**10.** Material nach Anspruch 1, dadurch gekennzeichnet, daß es der folgenden Formel entspricht:

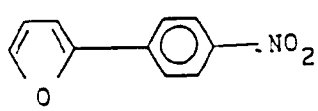

(Nitro-4-phenyl)-2-furan

**11.** Material nach Anspruch 1, dadurch gekennzeichnet, daß es der folgenden Formel entspricht:

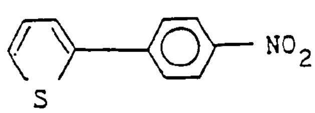

(Nitro-4-phenyl)-2-thiophen

**12.** Material nach Anspruch 1, dadurch gekennzeichnet, daß der heteroaromatische Zyklus bei 5 durch eine Gruppe substituiert wird, die ausgewählt wird unter $CH_3O$, $CH_3S$, $NH_2$, $CH_3NH$, $(CH_3)_2N$.

**13.** Verfahren zur Präparation des Materials nach Anspruch 8, dadurch gekennzeichnet, daß man das Zinkderivat

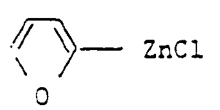

mit Parabromobenzonitril

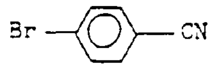

in Gegenwart von katalytischen Mengen von Palladiumkomplexen koppelt.

**14.** Verfahren zur Präparation des Materials nach Anspruch 9, dadurch gekennzeichnet, daß man das Magnesiumderivat

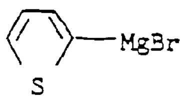

mit Paraiodobenzonitril

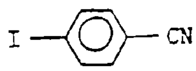

in Gegenwart von katalytischen Mengen von Palladiumkomplexen koppelt.

**15.** Verfahren zur Präparation des Materials nach Anspruch 10, dadurch gekennzeichnet, daß man das Zinkderivat

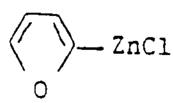

mit Paraiodonitronitril

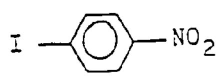

in Gegenwart von katalytischen Mengen von Palladiumkomplexen koppelt.

**16.** Verfahren zur Präparation des Materials nach Anspruch 11, dadurch gekennzeichnet, daß man das Zinkderivat

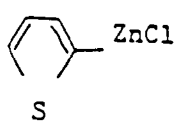

mit Paraiodonitrobenzen

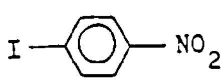

in Gegenwart von katalytischen Mengen von Palladiumkomplexen koppelt.

## Claims

1. An organic material possessing non-linear optical properties, and of which the molecule is formed:
   from an electron donor group comprising a heteroaromatic cycle whose chain includes 4 atoms of carbon and 1 atom selected from S, O, and N;
   from an electron attractor group selected from $NO_2$ and CN; and
   from an electron transmitter group selected from chains having n atoms of carbon (where $n < 4$) and including a double or a triple bond, and an aromatic system such as a phenyl ring, said electron transmitter group being bounded to said electron donor group by one of the said four atoms of carbon.

2. An organic material according to claim 1, characterized by the fact that the nitrogen atom is bonded to a radical R selected from H, $CH_3$, and $C_2H_5$.

3. A material according to claim 1 or 2, characterized by the fact that the said transmitter group is constituted by an ethenyl bond.

4. A material according to claim 3, characterized by the fact that it satisfies one of the following formulas:

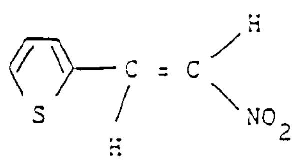

(transnitrovinylidene) - 2 thiophene

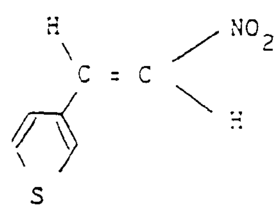

(transnitrovinylidene) - 3 thiophene

5. A material according to claim 3, characterized by the fact that it satisfies one of the following formulas:

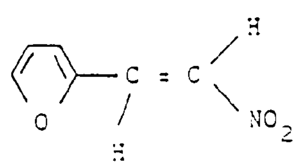

(transnitrovinylidene) - 2 furan

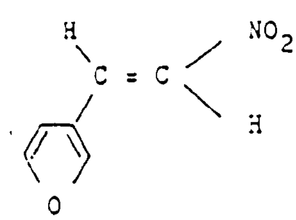

(transnitrovinylidene) - 3 furan

6. A material according to claim 3, characterized in that it satisfies one of the following formulas:

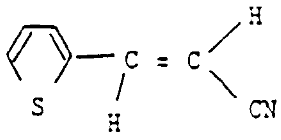

(transcyanovinylidene) - 2 thiophene

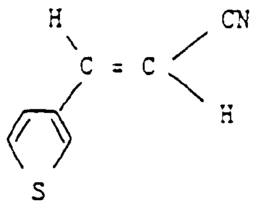

(transcyanovinylidene) - 3 thiophene

**7.** A material according to claim 3, characterized by the fact that it satisfies one of the following formulas:

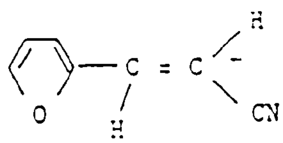

(transcyanovinylidene) - 2 furan

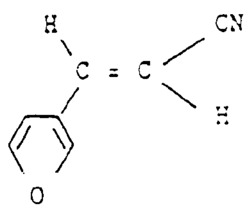

(transcyanovinylidene) - 3 furan

**8.** A material according to claim 1, characterized by the fact that it satisfies the following formula:

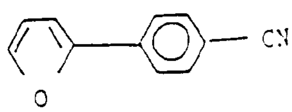

(cyano 4 phenyl) - 2 furan

**9.** A material according to claim 1, characterized by the fact that it satisfies the following formula:

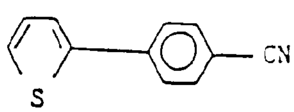

(cyano 4 phenyl) - 2 thiophene

**10.** A material according to claim 1, characterized by the fact that it satisfies the following formula:

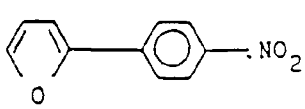

(nitro 4 phenyl) - 2 furan

**11.** A material according to claim 1, characterized by the fact that it satisfies the following formula:

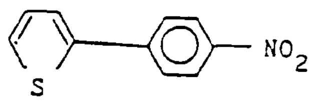

(nitro 4 phenyl) - 2 thiophene

**12.** A material according to claim 1, characterized by the fact that said heteroaromatic cycle is substituted at 5 by a group selected from: $CH_3O$, $CH_3S$; $NH_2$; $CH_3NH$; and $(CH_3)_2N$.

**13.** A method of preparing the material of claim 8, characterized by the fact that the zinc derivative

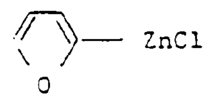

is coupled with parabromobenzonitrile

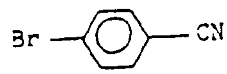

in the presence of catalytic quantities of palladium complexes.

**14.** A method of preparing material according to claim 9, characterized by the fact that the magnesium derivative

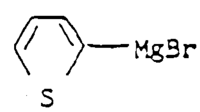

is coupled with paraiodobenzonitrile

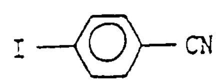

in the presence of catalytic quantities of palladium complexes.

**15.** A method of preparing the material of claim 10, characterized by the fact that the zinc derivative

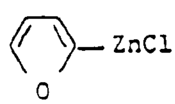

is coupled with paraiodonitrobenzene

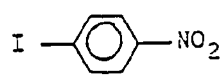

in the presence of catalytic quantities of palladium complexes.

**16.** A method of preparing the material of claim 11, characterized by the fact that the zinc derivative

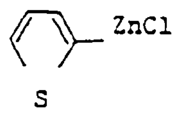

is coupled with paraiodonitrobenzene

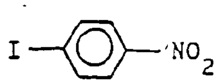

in the presence of catalytic quantities of palladium complexes.